# EUROPÄISCHE PATENTANMELDUNG

(11) **EP 1 591 540 A1**
(43) Veröffentlichungstag der Anmeldung: **02.11.2005**
(21) Anmeldenummer: 05009276.6
(22) Anmeldetag: 28.04.2005
(51) Int. Cl.: C12Q 1/68

(54) **Diagnose von Infektionserkrankungen**

(30) Priorität: 29.04.2004 DE 102004022350
(71) Anmelder: GenID Gmbh, 72479 Strassberg (DE)
(72) Erfinder: Schöllhorn, Volkmar, 72458 Albstadt (DE); Weilbach, Alfons, 72072 Tübingen (DE)
(74) Vertreter: Patentanwälte Ruff, Wilhelm, Beier, Dauster & Partner

(57) **Zusammenfassung**

Es wird ein Kit zur Diagnose von Infektionen, insbesondere von HPV (humaner Papillomavirus)-Infektionen, bereitgestellt. Dieser Kit enthält neben Mitteln zur Kontrolle der Qualität eines zervikalen Abstrichs Nachweismittel für Niedrigrisiko-HPV-Erreger und Nachweismittel für Hochrisiko-HPV-Erreger. Durch einen modularen Aufbau können mit dem erfindungsgemäßen Kit weitere Faktoren analysiert werden, um so das Risiko zur Entwicklung eines Zervixkarzinoms abschätzen zu können.

## Beschreibung

Die vorliegende Erfindung betrifft einen Kit zur Diagnose von Infektionserkrankungen, insbesondere von HPV (humaner Papillomavirus)-Infektionen, ein Verfahren zur Diagnose von Infektionserkrankungen und eine Verwendung des Kits zur Diagnose von Infektionen sowie ein Verfahren zur Kontrolle eines zervikalen Abstrichs.

Humane Papillomaviren (HPV) sind die häufigsten, sexuell übertragbaren, viralen Infektionserreger. Es ist davon auszugehen, da etwa 60 % der über 20jährigen Frauen mit HPV infiziert sind. Von diesen Infektionen heilen die meisten Fälle komplikationslos aus. Bei einem sehr geringen Anteil dieser Infektionen sind jedoch Komplikationen zu beobachten.

Abhängig vom Virustyp können Papillomavirus-Infektionen neben einer Vielzahl gutartiger Läsionen der Haut und der Schleimhäute auch zu einer der häufigsten malignen Erkrankungen der Frau, dem Zervixkarzinom, führen. Man geht davon aus, daß jede 10.000. HPV-Infektion sich zu einem Zervixkarzinom (Gebärmutterhalskrebs) entwickelt. Als Vorstufe eines Zervixkarzinoms läßt sich oftmals eine Neoplasie des Schleimhautgewebes, also eine Neubildung von Körpergewebe, als Vorstufe des malignen Tumors beobachten. In den USA beispielsweise registrierte man von allen diagnostizierten HPV-Infektionen etwa 55.000 Fälle einer Zervix-Neoplasie. Davon entwickelten etwa 15.000 Fälle ein Zervixkarzinom.

Eine spezifische antivirale Therapie gibt es bislang nicht, ebensowenig einen Impfstoff. Befallene Haut- bzw. Schleimhautbereiche können je nach Größe durch Laser, Kryotherapie oder Exzision behandelt werden. Bei malignen Formen der Erkrankung im Genitalbereich ist in der Regel eine operative Entfernung notwendig. Die frühzeitige Diagnose spielt dabei eine sehr große Rolle für die Prognose.

Über die zugrundeliegenden Pathomechanismen ist bisher nur relativ wenig bekannt. Es sind mittlerweile über 70 Virustypen und Subtypen der humanen Papillomaviren beschrieben. Hierbei lassen sich die Virustypen, die an Papillomavirus-Infektionen des Genitaltraktes beteiligt sind, zum einen in sogenannte Hochrisiko-Typen und zum anderen in sogenannte Niedrigrisiko-Typen einteilen. Als Hochrisiko-Typen werden die Virustypen eingestuft, die am häufigsten in zervikalen Karzinomen festgestellt werden. Andere Typen, die Niedrigrisiko-Typen, werden häufiger in gutartigen Läsionen nachgewiesen.

Um insbesondere bei einem auffälligen zytologischen Befund (Pap-Abstrich) eine Aussage zur Möglichkeit der Entwicklung eines malignen Tumors treffen zu können, wird bereits das Vorliegen von humanen Papillomaviren getestet. Weiterhin kann eine Typisierung dieser Viren vorgenommen werden, wobei nach Hochrisiko-Typen und Niedrigrisiko-Typen unterschieden wird. Zum Nachweis der HPV-Typen können Polymerasekettenreaktionen (PCR) durchgeführt werden, wobei bestimmte Primer eingesetzt werden, die zur Amplifizierung der DNA der jeweiligen Typen geeignet sind. Mit diesem Vorgehen kann also eine Aussage darüber getroffen werden, ob zum einen eine Infektion mit HPV vorliegt und zum anderen, ob der Typ des HPV-Erregers als Hochrisiko-Typ oder als Niedrigrisiko-Typ einzustufen ist. Bei einer Infektion mit einem Hochrisiko-Typ ist daher mit einer erhöhten Wahrscheinlichkeit zur Entwicklung eines Zervixkarzinoms zu rechnen. Es sind allerdings etwa 30 % der weiblichen Bevölkerung Trägerin von HPV-Erregern des Hochrisiko-Typs. Hiervon entwickelt nur ein äußerst geringer Prozentsatz wirklich ein Zervixkarzinom. Das bedeutet, daß durch die Einstufung als Trägerin von HPV-Erregern des Hochrisiko-Typs ein sehr beträchtlicher Anteil der Patientinnen zu Unrecht verunsichert wird. Diese herkömmliche Diagnostik ist daher sehr unspezifisch und als äußerst problematisch zu betrachten. Die Erfindung stellt sich demnach die Aufgabe, die Risikogruppe weiter einzugrenzen, um auf diese Weise eine klinisch wirklich relevante Prognose stellen zu können. Es soll daher ein Verfahren und ein Kit zur Diagnose von HPV-Infektionen und zur Abschätzung des Risikos zur Entwicklung eines Zervixkarzinoms bereitgestellt werden.

Diese Aufgabe wird durch einen Kit gemäß Anspruch 1 sowie durch ein Verfahren, wie es im Anspruch 10 ausgeführt ist, gelöst. Anspruch 19 betrifft die Verwendung eines erfindungsgemäßen Kits. Anspruch 20 befaßt sich mit einem Verfahren zur Kontrolle eines zervikalen Abstrichs. Bevorzugte Ausführungsformen dieser Erfindungsgegenstände sind in den abhängigen Ansprüchen beschrieben. Der Wortlaut sämtlicher Ansprüche wird hiermit durch Bezugnahme zum Inhalt der Beschreibung gemacht.

Der erfindungsgemäße Testkit ist zur Diagnose von Infektionen vorgesehen. Insbesondere können hiermit HPV-Infektionen näher charakterisiert werden. Der erfindungsgemäße Kit umfaßt zunächst Kontrollmittel, die die Qualität des zervikalen Abstrichs, welcher das Ausgangsmaterial für die Durchführung der Diagnose bildet, gewährleisten. Um wirklich aussagekräftige Aussagen machen zu können und um falsch-negative Diagnosen zu vermeiden, ist es erforderlich, daß mit dem Abstrich, der durch den Frauenarzt durchgeführt wird, ausreichend Material vom Gebärmutterhals entnommen wird. Gelangt beispielsweise durch einen unzureichenden Abstrich kein oder zu wenig humanes Zellmaterial, insbesondere Zellen oder Zellfragmente, zur weiteren Analyse, können die entsprechenden Erreger nicht nachgewiesen werden, und es kommt zu falsch-negativen Diagnosen. Um dieses Risiko auszuschließen, ist es erfindungsgemäß vorgesehen, daß entsprechende Kontrollmittel im Testkit enthalten sind, so daß nur dann eine Aussage über die HPV-Infektion bzw. die entsprechenden Erreger getroffen wird, wenn ausreichend Zellmaterial vorgelegen hat.

Darüber hinaus enthält der erfindungsgemäße Kit Nachweismittel für HPV-Erreger des Niedrigrisiko-Typs und Nachweismittel für HPV-Erreger des Hochrisiko-Typs. Mit Hilfe der Nachweismittel für die HPV-Erreger beider Typen können zum einen Aussagen getroffen werden, ob überhaupt eine HPV-Infektion vorliegt. Zum anderen können Aussagen darüber gemacht werden, ob bei dem vorliegenden Typ des Erregers mit einer höheren oder mit einer niedrigeren Wahrscheinlichkeit damit gerechnet werden kann, daß sich ein Zervixkarzinom entwickelt oder daß bereits ein solches vorliegt.

In einer besonders bevorzugten Ausführungsform des erfindungsgemäßen Kits handelt es sich bei den Kontrollmitteln für die Qualität des zervikalen Abstrichs um Nachweismittel, mit denen humane DNA nachgewiesen werden kann. Da DNA relativ fragil ist, stellt das Vorhandensein von humaner DNA sicher, daß im Abstrichmaterial wirklich humane Zellen oder Zellfragmente enthalten waren. Bevorzugterweise wird mit diesen Nachweismitteln die DNA des humanen Enzyms Glycerinaldehydphosphat-Dehydrogenase (GAPDH) nachgewiesen. Vorteilhafterweise werden hierfür bestimmte Primer eingesetzt, die mit den entsprechenden Abschnitten der DNA wechselwirken und mit deren Hilfe in einer sogenannten Polymerasekettenreaktion (PCR) diese Genabschnitte amplifiziert werden. Selbstverständlich können auch andere humane DNA-Abschnitte gewählt werden, die entsprechend nachgewiesen werden. Voraussetzung hierfür ist, daß die entsprechende DNA immer im humanen Zellgenom vorhanden ist, und daß sie spezifisch erkannt, insbesondere amplifiziert, werden kann. Andere Möglichkeiten zur Kontrolle des zervikalen Abstrichs sind beispielsweise der Nachweis von bestimmten humanen Proteinen oder beispielsweise der Nachweis von bestimmten enzymatischen Aktivitäten.

In einer bevorzugten Ausführungsform des erfindungsgemäßen Kits sind weiterhin Mittel enthalten, die zum Nachweis von Herpes simplex-Viren (HSV) geeignet sind. Hierbei ist der HSV II-Erreger von besonderem Interesse. Es ist bereits bekannt, daß der Nachweis von HSV II und HPV-Hochrisiko-Typ ein Hinweis für ein beträchtlich erhöhtes Risiko ist, ein Zervixkarzinom zu entwickeln. Erfindungsgemäß ist daher vorgesehen, neben der Qualitätskontrolle des Abstrichs und der Typisierung der HPV-Erreger zusätzlich zu testen, ob HSV II-Erreger vorhanden sind. Hierdurch kann die Risikogruppe der Patientinnen, welche möglicherweise ein Zervixkarzinom entwickeln werden, erheblich eingegrenzt werden. Durch den kombinierten Nachweis von HSV II und HPV-Hochrisiko-Typ können also wesentlich zuverlässigere Prognosen gestellt werden.

Darüber hinaus kann es vorteilhaft sein, daß ebenfalls getestet wird, ob HSV I-Erreger vorliegen. Bei dem Herpes simplex-Virus handelt es sich um einen sehr verbreiteten Virus, wobei hier insbesondere zwei Typen relevant sind. Lediglich beim zweiten Typ, HSV II, liegt in Kombination mit HPV-Hochrisiko-Typ ein erhöhtes Risiko vor, Gebärmutterhalskrebs zu entwickeln. HSV I ist in Bezug auf Gebärmutterhalskrebs bzw. auf die Entwicklung eines Zervixkarzinoms unproblematisch. Der Nachweis von HSV I erfolgt daher bevorzugt als Negativkontrolle. HSV I ist insbesondere dann ein wichtiges Ausschlußkriterium, wenn bekannt ist, daß eine Herpes-Infektion vorliegt und lediglich HSV I nachgewiesen werden kann. Wenn HSV II nicht nachgewiesen werden kann, kann aufgrund dieser Negativkontrolle mit Sicherheit ausgeschlossen werden, daß dieser besonders problematische Typ von HSV vorliegt. Folglich ist das Risiko bei einer Infektion mit HPV-Hochrisiko-Typ zur Entwicklung eines Zervixkarzinoms deutlich niedriger einzustufen.

Mit Hilfe dieser beschriebenen Nachweise kann bereits eine Einstufung des Risikos zur Entwicklung eines Zervixkarzinoms vorgenommen werden.

Stufe I: Nachweis von HPV-Niedrigrisiko-Typ (bei Abwesenheit von HPV-Hochrisiko-Typ). Es ist kein Risiko erkennbar, ein Zervixkarzinom zu entwickeln.

Stufe II: Nachweis von HPV-Hochrisiko-Typ. Es ist die Voraussetzung gegeben, ein Zervixkarzinom zu entwickeln. Das Risiko ist jedoch als äußerst gering einzustufen.

Stufe III: Nachweis von HSV II. Der zusätzliche Nachweis von HSV II in Kombination mit HPV-Hochrisiko-Typ erhöht beträchtlich das Risiko, ein Zervixkarzinom zu entwickeln. Insgesamt handelt es sich jedoch immer noch um eine nur sehr geringe Wahrscheinlichkeit.

Um die Risikogruppe noch weiter einschränken zu können, wird daher in einer weiteren bevorzugten Ausführungsform die Konzentration des HPV-Erregers vom Hochrisiko-Typ bestimmt. Diese sogenannte Viruslast erfolgt vorteilhafterweise durch Nachweis der Konzentrationsschwellen 10², 10⁴ und/oder 10⁶ Virusgenomkopien pro Probe und/oder Abstrich. Durch diese Bestimmung der Viruspartikelkonzentration können weitere Aussagen zur klinisch relevanten Veränderung des Zervixgewebes gemacht werden. Insbesondere zeigt ein Nachweis von lediglich 10² Virusgenomkopien pro Probe und/oder Abstrich, daß eine HPV-Infektion zwar vorhanden ist, aber klinisch normalerweise nicht relevant ist. Der Nachweis von 10⁴ bis 10⁶ Virusgenomkopien pro Probe und/oder Abstrich hingegen ist bereits bedeutsam und oftmals klinisch relevant. Patientinnen mit einer Virusbeladung in dieser Größenordnung zeigen sehr häufig morphologisch eine Neoplasie, also eine Neubildung von Gewebe, die in diesem Zusammenhang als Anomalie und als Vorstufe für ein cancerogen entartetes Gewebe einzustufen sein kann. Es ist damit zu rechnen, daß etwa 20 % dieser Patientengruppe ein Zervixkarzinom entwickeln werden bzw. bereits entwickelt haben. Bei einer Virusbeladung von 10⁴ bis 10⁶ Virusgenomkopien pro Probe und/oder Abstrich in Zusammenhang mit den anderen bereits beschriebenen Nachweisen ist also von einem hohen Risiko zur Entwicklung eines Zervixkarzinoms auszugehen. Diese Risikoeingruppierung läßt sich als Stufe IV beschreiben.

In einer weiteren bevorzugten Ausführungsform des erfindungsgemäßen Kits sind Nachweismittel vorgesehen, die einen weiteren Risikofaktor zur Entwicklung eines Zervixkarzinoms zeigen. Bei dieser letzten Stufe zur Untersuchung von HPV-Infektionen wird p16^{INK4a} getestet. Hierbei handelt es sich um ein Protein, welches als Onkogen von der humanen Zelle gebildet wird. Die DNA für p16^{INK4a} liegt im Chromosomensatz der humanen Zellen auch unter normalen Bedingungen vor. Sie wird jedoch nicht abgelesen und nicht zu einem Protein translatiert. Dies erfolgt erst, nachdem sich der Virus in die humane DNA integriert hat. Diese Integration des HPV in das Genom der humanen Zelle erfolgt immer an der gleichen Stelle, wobei das E2-Suppressorgen inaktiviert wird. Durch diesen Schritt wird die Bildung der Onkogene E6 und E7 induziert. Als Folge wird p16^{INK4a} mRNA gebildet. Das Vorliegen dieser mRNA bzw. des translatierten Proteins belegt daher die Integration des Virus in das Genom der menschlichen Zelle. Dieses Stadium ist als die unmittelbare Vorstufe eines Karzinoms zu betrachten. Es besteht also ein absolut a-kutes Risiko zur Entwicklung eines Zervixkarzinoms bzw. es kann sich bereits ein solches entwickelt haben. Dieser Nachweis läßt sich als Stufe V der Diagnose von HPV-Infektionen umschreiben und stellt die letzte Stufe des erfindungsgemäßen Kits zur Diagnose dieser Infektionen dar.

Der Nachweis von p16^{INK4a} kann über einen entsprechenden Proteinnachweis erfolgen. Dies kann beispielsweise mittels geeigneter Antikörper geschehen. Besonders bevorzugt ist es jedoch, wenn die mRNA für p16^{INK4a} nachgewiesen wird. Sie ist ein direktes Maß für das gebildete p16^{INK4a}-Protein. Vorzugsweise erfolgt der Nachweis der mRNA über geeignete PCR-Primer. Hierbei wird vorteilhafterweise ausgenutzt, daß bei der Bildung der mRNA Introns herausgesplict werden. Vorzugsweise wird daher zum Nachweis eine Sequenz gewählt, die nur in der gesplicten Form amplifizierbar ist. Auf diese Weise ist sichergestellt, daß die DNA für p16 ^{INK4a} nicht amplifiziert wird. Vorzugsweise wird über ein Intron hinweg unter Einsatz geeigneter Primer eine Sequenz von zwei Exons amplifiziert.

Für den Nachweis von p16^{INK4a}, insbesondere beim Nachweis der mRNA, wird vorzugsweise eine Kontrolle durchgeführt, die sicherstellt, daß das Ergebnis dieses Nachweises aussagekräftig ist. Als Kontrolle eignet sich hierfür in besonderer Weise der Nachweis von konstitutiv produzierter humaner mRNA. Eine geeignete Kontrollsequenz sollte also immer in einer gewissen Menge produziert werden, so daß gewährleistet ist, daß in der Probe Zellmaterial vorhanden ist, in welchem die Transkriptionsvorgänge in regulärer Weise ablaufen. Sollte dies nicht der Fall sein, hat auch das Ergebnis, daß keine p16^{INK4a} mRNA in der Probe vorhanden ist, keine Aussagekraft. Eine geeignete Kontrolle gewährleistet also, daß derartige falsch-negative Aussagen vermieden werden. Ein bevorzugtes Beispiel für eine geeignete Kontrolle ist der Nachweis von mRNA für β2-Mikroglobulin, welche immer entsprechend gesplict und konstitutiv transkribiert wird.

Diese Kontrollmessung von konstitutiv produzierter humaner mRNA sowie auch die oben beschriebene Kontrollmessung von humaner DNA hat den weiteren Vorteil, daß für die Konzentrationsbestimmung der Viruspartikel ein interner Standard herangezogen werden kann, so daß die ermittelte Virusmenge auf einen bestimmten Wert bezogen und objektiviert werden kann.

Der erfindungsgemäße Kit ist bevorzugterweise so aufgebaut, daß für die verschiedenen beschriebenen Nachweise und Kontrollen geeignete Primer eingesetzt werden, die für eine Amplifizierung der entsprechenden Nukleinsäuresequenzen über PCR-Reaktionen geeignet sind. Durch die Auswahl der entsprechenden Primer kann bestimmt werden, welche Nachweise und Kontrollen durchgeführt werden sollen. Vorteilhafterweise liegen hierbei die Primer als Gemische verschiedener Primer vor, so daß durch Auswahl eines bestimmten Gemisches entschieden wird, welche Nachweise geführt werden.

Weiterhin ist es bevorzugt, daß als Nachweismittel und/oder Kontrollmittel Gensonden in dem erfindungsgemäßen Kit enthalten sind, die spezifisch mit den nachzuweisenden Sequenzen wechselwirken können. In besonders vorteilhafter Weise sind diese Gensonden zum Nachweis von PCR-Produkten vorgesehen, die durch eine Amplifizierung mittels geeigneter Primer in der oben beschriebenen Weise eingesetzt werden können. In einer bevorzugten Ausführungsform sind diese Gensonden auf mindestens einem Träger fixiert, so daß die verschiedenen Nachweise ohne großen Aufwand durchgeführt werden können. Beispielsweise kann der Träger so ausgestaltet sein, daß es sich dabei um einen Teststreifen mit den entsprechend fixierten Gensonden handelt. Dieser Teststreifen kann beispielsweise mit dem PCR-Reaktionsgemisch benetzt werden, so daß die darin enthaltenen PCR-Produkte auf dem Teststreifen verteilt werden, beispielsweise durch Kapillarkräfte, und so zu den verschiedenen Gensonden gelangen und hier bei positiven Ergebnissen wechselwirken. Diese Wechselwirkungen werden vorzugsweise durch geeignete Methoden sichtbar gemacht. Dies kann beispielsweise durch eine Markierung der PCR-Produkte während der PCR-Reaktion geschehen, so daß bei Wechselwirkung eine Farbreaktion oder eine andere nachweisbare Reaktion an entsprechender Stelle des Trägers, insbesondere des Teststreifens, auftritt. Bezüglich des Nachweises der Virusbeladung kann beispielsweise für die nachzuweisenden Konzentrationsschwellen eine unterschiedliche Menge der jeweiligen Gensonde auf dem Träger fixiert sein, so daß nur bei dem Überschreiten einer bestimmten Konzentration eine Farbreaktion auf dem Träger an entsprechender Stelle auftritt.

Als weitere Möglichkeit für den Nachweis bestimmter Erreger können PCR-Primer in Kombination mit einer Sequenzierung der PCR-Produkte eingesetzt werden.

In einer bevorzugten Ausführungsform des erfindungsgemäßen Kits sind weiterhin Reagenzien zur Isolierung von DNA und/oder mRNA vorhanden. Hierbei handelt es sich beispielsweise um geeignete Puffer, Stammlösungen und/oder Enzyme, wie dem Fachmann geläufig ist. Entsprechende Reagenzien können selbstverständlich auch vom Anwender bereitgestellt werden.

In einer weiteren bevorzugten Ausführungsform sind im erfindungsgemäßen Kit Reagenzien zur Durchführung von PCR-Reaktionen enthalten. Hierbei kann es sich wiederum um geeignete Puffer, Stammlösungen und/oder Enzyme handeln, wie sich dem Fachmann erschließt. Weiterhin können auch geeignete Probengefäße und ähnliches im erfindungsgemäßen Kit enthalten sein.

Ein großer Vorteil des erfindungsgemäßen Kits zur Diagnose von Infektionen ist, daß dieser Kit modular aufgebaut ist und so eine stufenweise Diagnose der Infektionen möglich ist. So kann beispielsweise eine Typisierung des HPV-Erregers, der Nachweis für HSV II und die Bestimmung der Virusbeladung vorgenommen werden, ohne daß ein Nachweis von p16^{INK4a} erfolgt. Dies ist vor allem deshalb vorteilhaft, weil jede Patientin und jeder Patient ein Recht auf Nichtwissen besitzen sollte und beispielsweise die Möglichkeit haben sollte, nicht wissen zu wollen, ob eine Krebserkrankung oder eine unmittelbare Vorstufe davon vorliegt oder nicht. Dieser modulare Aufbau kann insbesondere so verwirklicht sein, daß verschiedene Primergemische zum Nachweis verschiedener Erreger- bzw. Kontrollkombinationen angeboten werden, wobei also jeweils ein bestimmtes Gemisch ausgewählt werden kann. Die Gensonden selbst können vorteilhafterweise immer in ihrer Gesamtheit auf einem Träger, beispielsweise auf einem Teststreifen, vorhanden sein. Bei Auswahl bestimmter Primer wird nur dann eine Farbreaktion bzw. eine nachweisbare Reaktion auftreten können, wenn die entsprechenden Primer ausgewählt wurden. Dies hat den Vorteil, daß immer der gleiche Träger eingesetzt werden kann. Der modulare Aufbau ist dann durch die verschiedenen Primergemische verwirklicht. Andererseits können auch unterschiedliche Träger mit verschiedenem Gensondenbesatz als modulare Komponenten bei immer gleicher Primerzusammensetzung eingesetzt werden. Hier sind alle Kombinationen möglich.

Die verschiedenen Primer können jeweils in einem Reaktionsgefäß bereitgestellt werden, so daß auch die PCR-Reaktionen im Gemisch ablaufen. Vorteilhafterweise sollten die Primer für Reaktionen mit mRNA und für Reaktionen mit DNA voneinander getrennt sein. Es kann jedoch auch bevorzugt sein, daß sämtliche Reaktionen in einem Gefäß ablaufen oder daß die verschiedenen Reaktionen getrennt voneinander durchgeführt werden.

In einer bevorzugten Ausführungsform des erfindungsgemäßen Kits sind als Zusatzmodul weiterhin Nachweismittel für den Erreger *Chlamydia trachomatis* enthalten. Die Infektionsrate mit Chlamydien ist bei jungen Frauen sehr hoch. Eine Chlamydieninfektion heilt aber oftmals ohne weitere Symptome wieder ab. Nichtsdestotrotz können Chlamydien sowohl bei Frauen als auch bei Männern eine Unfruchtbarkeit verursachen. Sie stellen daher mit HPV- und Herpes-Infektionen die drei relevanten sexuell übertragbaren Krankheiten (sexual transmitted diseases - STD) in den Industrieländern dar. Diese drei Erkrankungen machen zusammen weit über 90 % aller STDs aus. Von daher ist ein Nachweis von Chlamydien in Kombination mit Herpes-Erregern und HPV-Infektionen besonders interessant. Mit einem Nachweis dieser drei verschiedenen Erreger kann ein geeigneter Test für ein STD-Screening bereitgestellt werden. Ein solches Screening kann mit einer Probe aus einem Abstrich und mit einer bzw. zwei Kontrollen ohne großen Aufwand durchgeführt werden.

Als weitere Module für Extranachweise kommen vor allem der Nachweis von *Treponema pallidum* als Erreger der Syphillis und von *Neisseria gonorrhoe* als Erreger von Gonorrhoe (Tripper) in Betracht. Diese verschiedenen zusätzlichen Nachweise können in der oben beschriebenen Weise mit geeigneten Primern und Gensonden erfolgen. Es werden jedoch auch andere Nachweismethoden, beispielsweise über Antikörper oder Vergleichbares, von der Erfindung mit umfaßt. Bei den verschiedenen beschriebenen Nachweisen im Zusammenhang mit HPV-Infektionen sowie insbesondere bei den Zusatzmodulen (Chlamydien, *Treponema pallidum* und *Neisseria gonorrhoe)* sollte selbstverständlich der Patient immer entscheiden können, ob er entsprechende Nachweise durchgeführt haben möchte oder nicht.

Weiterhin umfaßt die Erfindung ein Verfahren zur Diagnose von Infektionen, insbesondere von HPV-Infektionen, wobei die Qualität eines zervikalen Abstrichs kontrolliert wird und das Abstrichmaterial auf HPV-Erreger des Niedrigrisiko-Typs und des Hochrisiko-Typs getestet wird. Zur Kontrolle der Abstrichsqualität wird insbesondere humane DNA nachgewiesen. Besonders vorteilhaft ist der Nachweis der DNA für das Enzym Glycerinaldehydphosphat-Dehydrogenase (GAPDH).

In einer bevorzugten Ausführungsform des erfindungsgemäßen Verfahrens wird weiterhin getestet, ob HSV II-Erreger vorhanden sind. Bevorzugterweise kann auch getestet werden, ob HSV I-Erreger vorliegen.

In einer weiteren bevorzugten Ausführungsform wird die Konzentration der Hochrisiko-HPV-Erreger nachgewiesen. Insbesondere werden die Konzentrationsschwellen 10², 10⁴ und/oder 10⁶ Virusgenomkopien pro Probe und/oder Abstrich analysiert, da bei diesen Konzentrationsschwellen der diagnostische Aussagegehalt besonders relevant ist.

In einer weiteren bevorzugten Ausführungsform des Verfahrens wird p16^{INK4a} untersucht. Besonders bevorzugt ist es, die mRNA zu testen. p16^{INK4a} stellt einen Marker für die Integration der Virus-DNA in die humane DNA dar. Bei einem positiven Nachweis von p16^{INK4a} ist daher davon auszugehen, daß der Virus bereits integriert ist und daß eine unmittelbare Vorstufe eines Karzinoms besteht. Vorteilhafterweise wird bei Durchführung des Tests auf p16^{INK4a} mRNA kontrolliert, ob konstitutiv produzierte humane mRNA vorliegt.

Das erfindungsgemäße Verfahren ist vorteilhafterweise dadurch gekennzeichnet, daß DNA und/oder mRNA aus der Abstrichsprobe isoliert wird. Die Nachweise der verschiedenen beschriebenen Parameter erfolgen bevorzugterweise mit geeigneten Primern. Diese Primer werden vor allem zur Durchführung von PCR-Reaktionen eingesetzt, um so die entsprechenden Sequenzen zu amplifizieren und einer weiteren Analyse leicht zugänglich zu machen. Die Analyse kann vorteilhafterweise mit Gensonden durchgeführt werden, wobei insbesondere diese Gensonden eingesetzt werden, um die entsprechenden PCR-Produkte nachzuweisen.

In einer weiteren bevorzugten Ausführungsform des erfindungsgemäßen Verfahrens wird zusätzlich der Erreger *Chlamydia trachomatis* getestet. In weiteren bevorzugten Ausführungsformen können zusätzlich oder alternativ dazu der Erreger *Treponema pallidum* und/oder der Erreger *Neisseria gonorrhoe* untersucht werden. Bezüglich der verschiedenen Merkmale und Ausführungsformen des erfindungsgemäßen Verfahrens wird auf die obige Beschreibung verwiesen.

Weiterhin umfaßt die Erfindung eine Verwendung des beschriebenen Kits zur Diagnose von Infektionen, insbesondere von HPV-Infektionen. Hierfür wird vorzugsweise als Ausgangsmaterial ein zervikaler Abstrich eingesetzt. Bezüglich weiterer Merkmale dieser Verwendung wird auf die obige Beschreibung verwiesen.

Schließlich umfaßt die Erfindung ein Verfahren zur Kontrolle eines zervikalen Abstrichs, wobei humane DNA nachgewiesen wird. In besonders vorteilhafter Weise wird hierbei die DNA der GAPDH analysiert. Unabhängig von den durchzuführenden Tests, die mit zervikalen Abstrichen durchgeführt werden, ist die Qualität des Abstrichs entscheidend. Voraussetzung für aussagekräftige Testergebnisse ist in der Regel, daß im Abstrich humane Zellen oder Zellfragmente enthalten sind. Erfindungsgemäß wird daher gemessen, ob humane DNA im Abstrichmaterial vorhanden ist. Auf diese Weise werden falsch-negative Diagnosen vermieden. Beispielsweise kann ein entsprechender Nachweis mittels geeigneter Primer über eine PCR-Reaktion erfolgen. Die entstehenden PCR-Produkte können vorteilhafterweise mit Hilfe von geeigneten Gensonden nachgewiesen werden.

Weitere Merkmale der Erfindung ergeben sich aus den Unteransprüchen in Kombination mit den Ausführungsbeispielen. Hierbei können die verschiedenen Merkmale jeweils für sich oder in Kombination miteinander verwirklicht sein.

In den Abbilungen zeigen
Fig. 1: Beispiel eines Teststreifens mit verschiedenen Gensonden.
Fig.2: Mögliche Testergebnisse der Teststreifen aus Fig. 1.

### Material und Methoden:

### Primer und Sonden:

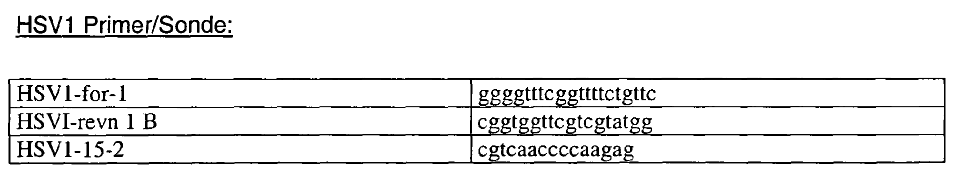

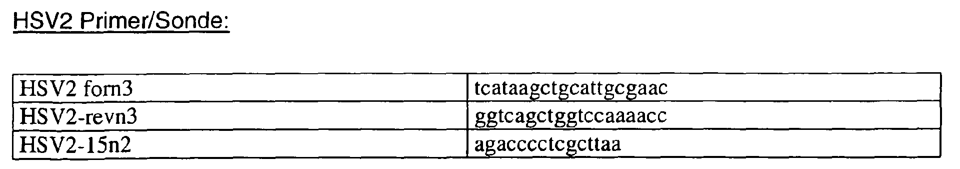

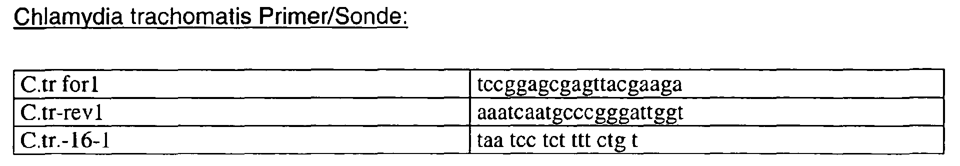

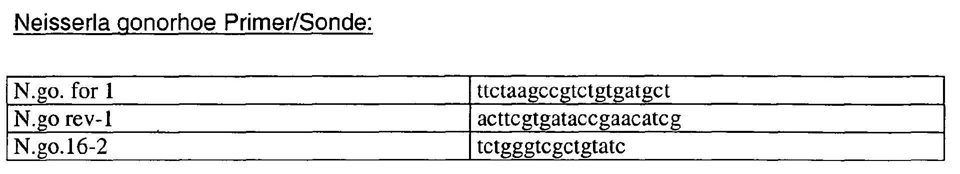

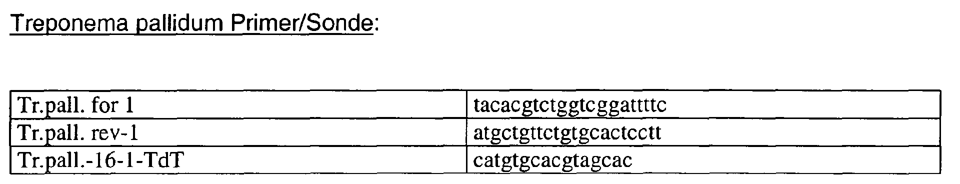

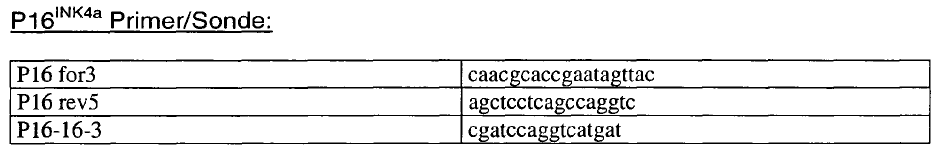

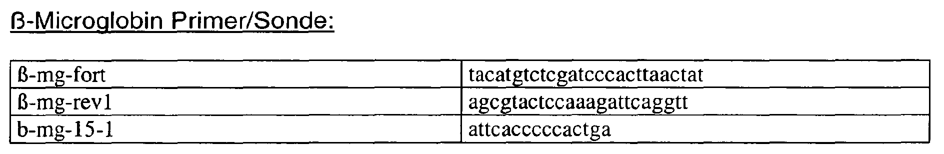

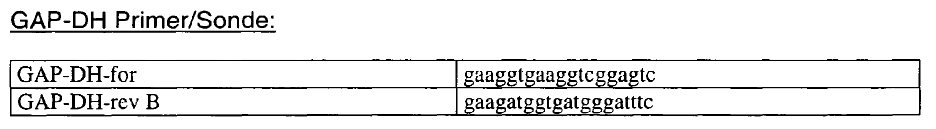

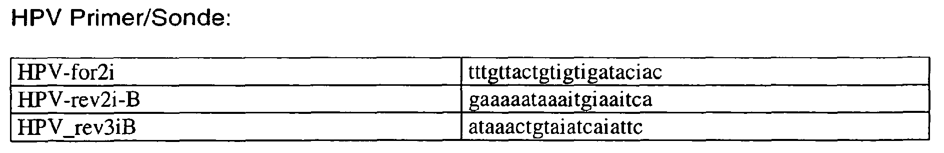

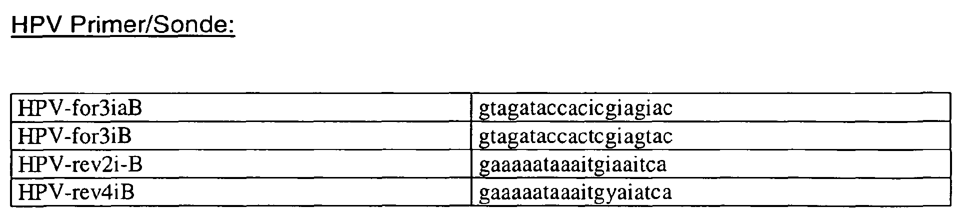

### Zuordnung der HPV-Primer:

Mittels der Primer erfolgt noch keine Differenzierung nach HPV-Typen. Die Primer gewährleisten jedoch, dass möglichst alle relevanten HPV-Genotypen detektiert werden. Durch die Reverse Hybridisierung erfolgt die Differenzierung der HPV-Typen in high risk und low risk.

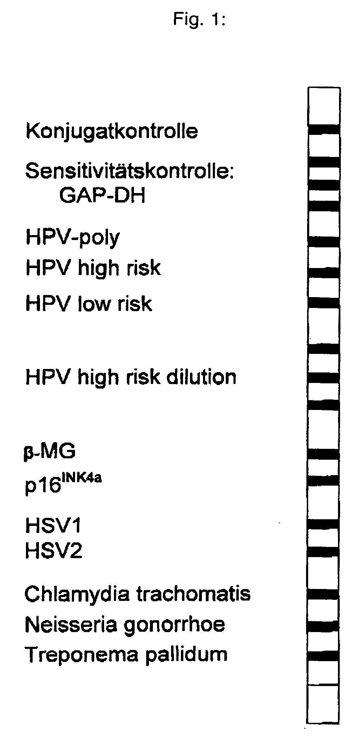

Figur 1 zeigt ein Beispiel eines möglichen Aufbaus eines Teststreifens mit entsprechenden Gensonden, auf denen die aufgearbeiteten Proben aufgetragen werden können, so dass diese mit den entsprechenden Sequenzen interagieren. Zu erkennen sind zum einen die Konjungatkontrolle und die Sensitivitätskontrolle (GAP-DH). Ferner sind einzelne Gensonden für HPV-poly, HPV-high risk, HPV-low risk und HPV-high risk dilution zu erkennen sowie die (fakultativ vorhandenen) Gensonden für die Erreger (Chlamydia trachomatis, Neisseria gonorrhoe, Treponema pallidum HSV1, HSV2) sowie die Gensonden für β-Microglobin und P16^{INK4a}. Solch ein Teststreifen erlaubt die nähere Charakterisierung der HPV-Infektion.

Eine mögliche Einteilung der PN-Mixe/Module kann wie folgt aussehen:

| PN-Mix | Nachweis | Material | Methode |
|---|---|---|---|
| A | GAP-DH | DNA | PCR |
| B I | HPV | DNA | PCR |
| B II | HPV | DNA | PCR |
| C | P16^{INK4a} | MPNA | Rt-PCR |
| D | HSV1+2, C.tr. | DNA | PCR |
| E | N.go., Tr.pall. | DNA | PCR |

### Probenaufarbeitung:

Abstrich- beziehungsweise Biopsiematerial wird in einem geeigneten kommerziell erhältlichen Transportmedium, in dem DNA und RNA intakt bleiben, angeliefert. Die DNA beziehungsweise RNA-Extraktion (für das Modul P19^{INK4a}) erfolgt über allgemein übliche Säulenreinigungsverfahren. Die gewonnene DNA beziehungsweise RNA wird in die PCR mit den jeweiligen PN-Mixen der verschiedenen Module eingesetzt.

### Testdurchführung:

1. Die Pufferlösungen werden auf 47°C vorgewärmt, alle anderen Lösungen auf Raumtemperatur angewärmt. Inkubationswannen entsprechend der Anzahl der Proben und der Kontrollen werden vorbereitet. Vertiefungen, die benutzt werden sollen, werden am Rand markiert. Bereits gebrauchte Kavitäten werden nicht noch einmal verwenden.
2. In die markierten Kavitäten werden je 40 µl Denaturierungsreagenz pipettiert.
3. Je 20 µl Amplifikat von jedem der zu untersuchenden PCR-Ansätze werden zu dem Tropfen Denaturierungsreagenz pipettiert, gut gemischt und 5 Minuten bei Raumtemperatur inkubiert.
4. Es wird jeweils vorsichtig 1 ml vorgewärmte und gemischte Hybridisierungspuffer hinzugeben.
5. Die einzelnen Streifen werden mit einer Pinzette aus dem Röhrchen entnommen und in die Inkubationswanne gelegt. Die Streifen müssen vollständig mit Flüssigkeit bedeckt sein und die beschichtete Seite soll nach oben weisen. Dies gilt auch für alle nachfolgenden Inkubations-und Waschschritte. Streifen, die sich z. B. durch Zugabe von Waschpuffer wenden, werden mit einer Pinzette an einem Ende aufgenommen und zurückgedreht.
6. Die Wanne wird für 30 min. bei 47°C im Schüttelwasserbad inkubiert (Frequenz beachten).
7. Hybridisierungspuffer wird vollständig abgegossen und die Streifen anschließend zweimal 1 Minute mit je 1 ml vorgewärmter und gemischter Stringent-Waschlösung bei Raumtemperatur auf dem Horizontalschüttler (Frequenz!) gewaschen.
8. Jeweils 1 ml vorgewärmte Stringent-Waschlösung wird zugegeben und 15 Minuten bei 47°C im Wasserbad unter Schütteln inkubiert.
9. Von diesem Schritt an wird bei Raumtemperatur gearbeitet. Die Lösung wird abgeklopft und die Streifen zweimal mit je 1 ml Waschlösung je 1 Minute gewaschen (Schüttler).
10. 1 ml vorbereitetes Konjugat (Konjugat-Konzentrat im Verhältnis 1:100 mit Konjugat-Puffer verdünnt) wird zu jedem Streifen gegeben und 30 Minuten auf dem Horizontalschüttler inkubiert.
11. Das Konjugat wird entfernt und dreimal je 1 Minute mit je 1 ml Waschlösung gewaschen (Horizontalschüttler).
12. Je 1 ml auf Raumtemperatur erwärmtes Substrat wird in die Kavitäten pipettiert und, je nachdem wie rasch die Farbreaktion abläuft, zwischen 10 und 20 Minuten auf dem Horizontalschüttler inkubiert.
13. Die Reaktion wird durch zweimaliges Waschen mit destilliertem Wasser gestoppt. Die Streifen werden mit einer Pinzette aus den Kavitäten entnommen und zum Trocknen auf Filterpapier gelegt.

Die getrockneten Streifen werden anschließend ausgewertet und lichtgeschützt aufbewahrt.

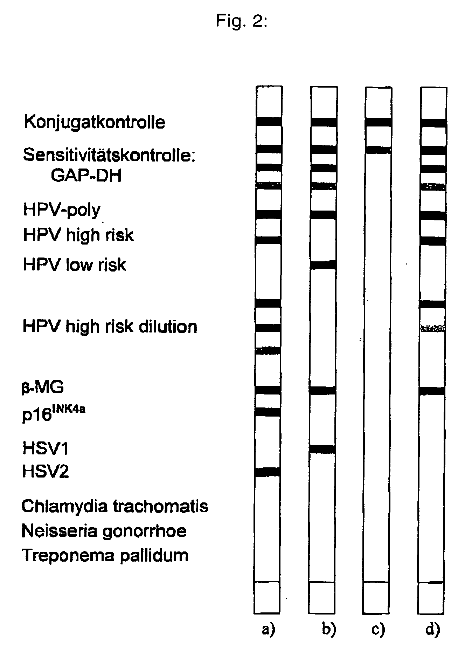

Figur 2 zeigt ein mögliches Testergebnis. In Figur 2a) wird ein mögliches Ergebnis gezeigt, bei dem eine HPV-high risk Infektion mit einer hohen Viruslast vorliegt. Solch ein Testergebnis würde einen Hinweis darauf liefern, dass eine Virusintegration und eine zusätzliche HSV2 Infektion bei dem Probanten vorliegt. Figur 2b) zeigt ein mögliches Testergebnis bei einer HPV-low risk Infektion bei einer gleichzeitigen Koinfektion mit HSV1. Figur 2c) zeigt ein Testergebnis, bei dem die GAP-DH Kontrolle nur sehr schwach entwickelt ist, was auf zu wenig DNA schließen lässt in dem Probenmaterial schließen lässt. Solch ein Testergebnis würde nicht ausgewertet werden, da das Probenmaterial offensichtlich zu wenige Zellen und/oder zu wenig PCR-Hemmstoff enthielt. Figur 2d) zeigt ein mögliches Testergebnis bei einer HPV-high risk Infektion mit einer moderaten bis geringen Viruslast. Solch ein Testergebnis würde auf ein geringes Risiko eines Zervixkarzinoms schließen lassen.

## Patentansprüche

1. Kit zur Diagnose von Infektionen, insbesondere von HPV (humaner Papillomavirus)-Infektionen, umfassend
- Mittel zur Kontrolle der Qualität eines zervikalen Abstrichs,
- Nachweismittel für Niedrigrisiko-HPV-Erreger und
- Nachweismittel für Hochrisiko-HPV-Erreger.

2. Kit nach Anspruch 1, **dadurch gekennzeichnet, daß** als Mittel zur Kontrolle des zervikalen Abstrichs Nachweismittel für humane DNA, insbesondere für die DNA der GAPDH (Glycerinaldehydphosphat-Dehydrogenase), enthalten sind.

3. Kit nach Anspruch 1 oder Anspruch 2, **dadurch gekennzeichnet, daß** weiterhin enthalten sind
- Nachweismittel für HSV I (Herpes simplex Virus)-Erreger und/oder
- Nachweismittel für HSV II-Erreger.

4. Kit nach einem der vorhergehenden Ansprüche, **dadurch gekennzeichnet, daß** weiterhin enthalten sind
- Mittel zum Nachweis der Konzentration von Hochrisiko-HPV-Erregern, insbesondere zum Nachweis der Konzentrationsschwellen 10², 10⁴ und/oder 10⁶ 10⁶ Virusgenomkopien pro Probe und/oder Abstrich.

5. Kit nach einem der vorhergehenden Ansprüche, **dadurch gekennzeichnet, daß** weiterhin enthalten sind
- gegebenenfalls Mittel zur Kontrolle von konstitutiv produzierter humaner mRNA und
- Nachweismittel für p16 ^{INK4a}, insbesondere für p16 ^{INK4a} mRNA.

6. Kit nach einem der vorhergehenden Ansprüche, **dadurch gekennzeichnet, daß** als Nachweismittel und/oder Kontrollmittel Primer für PCR-Reaktionen enthalten sind, wobei vorzugsweise die Primer als Gemische verschiedener Primer enthalten sind.

7. Kit nach einem der vorhergehenden Ansprüche, **dadurch gekennzeichnet, daß** als Nachweismittel und/oder Kontrollmittel Gensonden enthalten sind, wobei vorzugsweise die Gensonden zum Nachweis von PCR-Produkten vorgesehen sind.

8. Kit nach Anspruch 8, **dadurch gekennzeichnet, daß** die Gensonden auf mindestens einem Träger, insbesondere mindestens einem Teststreifen, fixiert sind.

9. Kit nach einem der vorhergehenden Ansprüche, **dadurch gekennzeichnet, daß** weiterhin enthalten sind
- Reagenzien zur Isolierung von DNA und/oder mRNA, und/oder
- Reagenzien zur Durchführung von PCR-Reaktionen, und/oder
- Nachweismittel für den Erreger *Chlamydia trachomatis,* und/oder
- Nachweismittel für den Erreger *Treponema pallidum* und/ oder
- Nachweismittel für den Erreger *Neisseria gonorrhoe.*

10. Verfahren zur Diagnose von Infektionen, insbesondere von HPV-Infektionen, umfassend die Verfahrensschritte
- Kontrollieren der Qualität eines zervikalen Abstrichs,
- Testen auf Niedrigrisiko-HPV-Erreger und
- Testen auf Hochrisiko-HPV-Erreger.

11. Verfahren nach Anspruch 10, **dadurch gekennzeichnet, daß** zum Kontrollieren der Qualität des zervikalen Abstrichs humane DNA, insbesondere die DNA der GAPDH, nachgewiesen wird.

12. Verfahren nach Anspruch 10 oder Anspruch 11, **dadurch gekennzeichnet, daß** weiterhin durchgeführt wird
- Testen auf HSV I-Erreger und/oder
- Testen auf HSV II-Erreger.

13. Verfahren nach einem der Ansprüche 10 bis 12, **dadurch gekennzeichnet, daß** weiterhin durchgeführt wird
- Nachweisen der Konzentration von Hochrisiko-HPV-Erregern, insbesondere Nachweisen der Konzentrationsschwellen 10², 10⁴ und/oder 10⁶ Virusgenomkopien pro Probe und/oder Abstrich.

14. Verfahren nach einem der Ansprüche 10 bis 13, **dadurch gekennzeichnet, daß** weiterhin durchgeführt wird
- gegebenenfalls Kontrollieren von konstitutiv produzierter humaner mRNA und
- Testen auf p16 ^{INK4a}, insbesondere auf p16 ^{INK4a} mRNA.

15. Verfahren nach einem der Ansprüche 10 bis 14, **dadurch gekennzeichnet, daß** DNA und/oder mRNA aus dem zervikalen Abstrich isoliert wird.

16. Verfahren nach einem der Ansprüche 10 bis 15, **dadurch gekennzeichnet, daß** PCR-Reaktionen mit geeigneten Primern durchgeführt werden.

17. Verfahren nach einem der Ansprüche 10 bis 16, **dadurch gekennzeichnet, daß** Gensonden eingesetzt werden, wobei vorzugsweise die Gensonden zum Nachweisen von PCR-Produkten eingesetzt werden.

18. Verfahren nach einem der Ansprüche 10 bis 17, **dadurch gekennzeichnet, daß** weiterhin durchgeführt wird
- Testen auf den Erreger *Chlamydia trachomatis*, und/oder
- Testen auf den Erreger *Treponema pallidum*, und/oder
- Testen auf den Erreger *Neisseria gonorrhoe.*

19. Verwendung eines Kits gemäß einem der Ansprüche 1 bis 9 zur Diagnose von Infektionen, insbesondere von HPV-Infektionen, wobei vorzugsweise als Ausgangsmaterial ein zervikaler Abstrich eingesetzt wird.

20. Verfahren zur Kontrolle eines zervikalen Abstrichs, **dadurch gekennzeichnet, daß** humane DNA nachgewiesen wird, insbesondere die DNA der GAPDH.

21. Verfahren nach Anspruch 20, **dadurch gekennzeichnet, daß** zum Nachweis der DNA mindestens eine PCR-Reaktion mit geeigneten Primern durchgeführt wird, wobei vorzugsweise entstehende PCR-Produkte mit Gensonden nachgewiesen werden.
